# EUROPEAN PATENT SPECIFICATION

(11) **EP 4 038 041 B1**
(45) Date of publication and mention of the grant of the patent: **04.12.2024**
(21) Application number: 20789743.0
(22) Date of filing: 23.09.2020
(51) Int. Cl.: C07C 41/06, C07C 43/10, C07C 43/13, B01J 29/70

(54) **METHODS OF ETHERIFICATION**
VERFAHREN ZUR VERETHERUNG
PROCÉDÉS D'ÉTHÉRIFICATION

(30) Priority: 30.09.2019 US 201962907801 P
(43) Date of publication of application: 10.08.2022
(73) Proprietor: Dow Global Technologies LLC, Midland, MI 48674 (US)
(72) Inventor: LEE, Wen-Sheng, Midland, Michigan 48667 (US); KILOS, Beata A., Midland, Michigan 48667 (US); KU, Sung-Yu, Freeport, Texas 77566 (US); KING, Stephen W., League City, Texas 77573 (US)
(74) Representative: Beck Greener LLP
(86) International application number: PCT/US2020/052095
(87) International publication number: WO 2021/067077

(56) References cited:
- EP-A2- 0 846 671
- WO-A1-2013/146370
- JP-A- 2000 300 994
- US-A1- 2009 240 086
- US-B2- 7 807 615

## Description

### Field of Disclosure

The invention is defined in the claims. Embodiments of the present disclosure are directed towards methods of etherification, more specifically, embodiments are directed towards methods of etherification including reducing templates of a zeolite catalyst to provide a reduced template zeolite catalyst and contacting the reduced template zeolite catalyst with an olefin and an alcohol to produce a monoalkyl ether.

### Background

Monoalkyl ethers are useful for a number of applications such as solvents, surfactants, and chemical intermediates, for instance. There is continued focus in the industry on developing new and improved materials and/or methods that may be utilized for making monoalkyl ethers.

JP2000-300994 discloses a catalyst which is an inorganic solid acid containing a Group 8 metal element, preferably a crystalline metallosilicate, for producing (poly)alkylene glycol monoalkyl ether, which has a long catalyst life even if it is regenerated, and a method for using the catalyst.

EP0846671 discloses a process for producing a (poly)alkylene glycol monoalkyl ether with high selectivity and high yield, in which the (poly)alkylene glycol monoalkyl ether is produced by reacting an olefin and a (poly)alkylene glycol in the presence of a catalyst, wherein either: 1. a crystalline metallosilicate is used as the catalyst, at least a portion of the used catalyst is regenerated, and the regenerated catalyst is recycled as the catalyst for the reaction; or 2. the reaction between the olefin and the (poly)alkylene glycol is carried out in the presence of either or both of a (poly)alkylene glycol dialkyl ether and an alcohol.

US2009/240086 discloses a process for making glycerol di-tert-butyl ethers, in which glycerol and isobutylene are reacted in the presence of a beta-zeolite having a silicon to aluminium ratio greater than 150, and/or in which the etherification is performed in the presence of a beta-zeolite and added tert-butyl alcohol. The processes provide glycerol di-tert-butyl ethers while reducing the generation of isobutylene dimers and trimers.

WO2013/146370 discloses a process of making 3-alkoxy-3-methyl-1-butanol using at least one specific zeolite catalyst selected from beta-zeolite and Y-zeolite when reacting IPEA (3-methyl-3-buten-1-ol) or PNA (3-methyl-2-buten-1-ol) with a primary alcohol having 1 to 5 carbon atoms.

US7807615 discloses a process for preparing polyol alkyl ethers by reacting compounds having at least three hydroxyl functionalities with olefins in the presence of acidic catalysts at a pressure of from 0.5 to 10 bar and a temperature of from 20 to 250 °C such that at least one of the hydroxyl functionalities is not reacted but the rest are replaced by an alkoxyl moiety.

### Summary

The present disclosure provides methods of etherification, the methods including reducing templates of a zeolite catalyst to provide a reduced template zeolite catalyst having from 3 to 15 weight percent of templates maintained following calcination of the zeolite catalyst; and contacting the reduced template zeolite catalyst with an olefin and an alcohol to produce a monoalkyl ether, wherein the zeolite catalyst is a zeolite beta catalyst, and wherein the alcohol is a (poly)alkylene glycol.

The above summary of the present disclosure is not intended to describe each disclosed embodiment or every implementation of the present disclosure. The description that follows more particularly exemplifies illustrative embodiments. In several places throughout the application, guidance is provided through lists of examples, which examples can be used in various combinations. In each instance, the recited list serves only as a representative group and should not be interpreted as an exclusive list.

### Detailed Description

Methods of etherification are disclosed herein. The methods include reducing templates of a zeolite catalyst to provide a reduced template zeolite catalyst and contacting the reduced template zeolite catalyst with an olefin and an alcohol to produce a monoalkyl ether, wherein the zeolite catalyst is a zeolite beta catalyst, and wherein the alcohol is a (poly)alkylene glycol.

Advantageously, the methods of etherification disclosed herein can provide an improved, i.e. greater, monoalkyl ether selectivity, as compared to etherifications that do not utilize the reduced template zeolite catalyst, as discussed further herein. Improved monoalkyl ether selectivity can be desirable for a number of applications, such as utilization as a chemical intermediate. As an example, the monoalkyl ether may be utilized in a surfactant production by ethoxylation process, where the monoalkyl ether can desirably influence the surfactant's properties, e.g. as compared to a dialkyl ether.

Additionally, the methods of etherification disclosed herein can provide an improved, i.e. lesser, dialkyl ether selectivity, as compared to etherifications that do not utilize the reduced template zeolite catalyst, as discussed further herein. The improved, lesser, dialkyl ether selectivity can be desirable for a number of applications, such as a surfactant production by ethoxylation process, where the dialkyl ether can undesirably influence the surfactant's properties, e.g. as compared to a monoalkyl ether. In other words, dialkyls are an undesirable product.

Zeolite catalysts are crystalline metallosilicates, e.g., aluminosilicates, constructed of repeating TO₄ tetrahedral units where T may be Si, Al or P (or combinations of tetrahedral units), for example. These units are linked together to form frameworks having regular intra-crystalline cavities and/or channels of molecular dimensions, e.g., micropores.

Embodiments of the present disclosure provide that the zeolite catalyst is a synthetic zeolite catalyst. Synthetic zeolite catalysts can be made by a known process of crystallization of a silica-alumina gel in the presence of alkalis and templates, for instance. Examples of synthetic zeolite catalysts include zeolite beta catalysts (BEA), Linde Type A (LTA), Linde Types X and Y (Al-rich and Si-rich FAU), Silicalite-1, ZSM-5 (MFI), Linde Type B (zeolite P), Linde Type F (EDI), Linde Type L (LTL), Linde Type W (MER), and SSZ-32 (MTT) as described using IUPAC codes in accordance with nomenclature by the Structure Commission of the International Zeolite Association.. IUPAC codes describing Crystal structures as delineated by the Structure Commission of the International Zeolite Association refer to the most recent designation as of the priority date of this document unless otherwise indicated.

The present invention provides that the zeolite catalyst is a zeolite beta (BEA) catalyst. One or more embodiments provide that the zeolite catalyst includes a number of Bronsted acid sites, i.e., sites that donate protons.

The zeolite catalyst can have a SiO₂/Al₂O₃ mole ratio from 5:1 to 1500:1 as measured using Neutron Activation Analysis. All individual values and subranges from 5:1 to 1500:1 are included; for example, the zeolite catalyst can have a SiO₂/Al₂O₃ mole ratio from a lower limit of 5:1, 10:1, 15:1, or 20:1 to an upper limit of 1500: 1, 750:1, 300:1, or 100:1.

The zeolite catalyst can have a mean pore diameter from 5 to 12 angstroms. All individual values and subranges from 5 to 12 angstroms are included; for example, the zeolite catalyst can have a mean pore diameter from a lower limit of 5 or 7 angstroms to an upper limit of 11 or 12 angstroms.

The zeolite catalyst can have surface area from 130 to 1000 m²/g. All individual values and subranges from 130 to 1000 m²/g are included; for example, the zeolite catalyst can have a surface area from a lower limit of 130, 150, 175, 300, 400, or 500 m²/g to an upper limit of 1000, 900, or 800 m²/g. Surface area is measured according to ASTM D4365 - 19.

As mentioned, the zeolite catalyst is made by a process that utilizes a template, which may also be referred to as an organic template. Templates may also be referred to as templating agents and/or structure-directing agents (SDAs). The template can be added to the reaction mixture for making the zeolite catalyst to guide, e.g., direct, the molecular shape and/or pattern of the zeolite catalyst's framework. When the zeolite catalyst making process is completed, the zeolite catalyst includes templates, e.g., templates located in the micropores of the zeolite catalyst. Templates are utilized in the formation of the zeolite catalyst. One or more embodiments provides that the template comprises ammonium ions. Zeolite catalyst that include templates can be made by known processes. Zeolite catalyst that include templates can be obtained commercially. Examples of suitable commercially available metallosilicate catalysts include CP814E, CP814C, CP811C-300, CBV 712, CBV 720, CBV 760, CBV 2314, CBV 10A from ZEOLYST INTERNATIONAL^{™} of Conshohocken, PA.

Various templates that may be utilized for making zeolite catalysts are known. Examples of templates include tetraethylammonium hydroxide; N,N,N-trimethyl-1-adamante-ammonium hydroxide; hexamethyleneimine; and dibenzylmethylammonium; among others.

As mentioned, the methods disclosed herein include reducing templates of a zeolite catalyst to provide a reduced template zeolite catalyst. The present invention provides that templates of the zeolite catalyst are reduced by calcination. The present invention provides that not all of the templates of the zeolite catalyst are removed by calcination.

To reduce templates, the zeolite catalyst may be calcined at temperature from 300 °C to 510 °C. All individual values and subranges from 300 °C to 510 °C are included; for example, the zeolite catalyst may be calcined at from a lower limit of 300 °C, 310 °C, or 315 °C to an upper limit of 510 °C, 505 °C, or 500 °C.

To reduce templates, the zeolite catalyst may be calcined in a number of known calcination environments. For instance, the zeolite catalyst may be calcined in an air environment or a nitrogen environment.

To reduce templates, the zeolite catalyst may be calcined, i.e., exposed to a temperature from 300 °C to 510 °C in a calcination environment, from 1 hour to 24 hours. All individual values and subranges from 1 hour to 24 hours are included; for example, the zeolite catalyst may be calcined at from a lower limit of 1 hour, 3 hours, or 6 hours to an upper limit of 24 hours, 18 hours, or 12 hours.

The present invention provides that a weight of the zeolite catalyst is reduced by calcination to provide a reduced template zeolite catalyst. The weight of the zeolite catalyst is reduced by calcination by reducing templates, i.e., by removing some templates from the zeolite catalyst. The weight of the zeolite catalyst can be reduced from 5 weight percent to 15 weight percent based upon a total weight of the initial zeolite catalyst and templates. All individual values and subranges from 5 weight percent to 15 weight percent are included; for example, the weight of the zeolite catalyst can be reduced from a lower limit of 5, 5.3, or 5.5 weight percent to an upper limit of 15, 14.7, or 14.5 weight percent based upon a total weight of the zeolite catalyst and templates. For instance, if a zeolite catalyst weighed 90 grams and templates included therein weighed 10 grams, and the zeolite catalyst was calcined to reduce templates such that the reduced template zeolite catalyst weighed 90 grams and templates included therein weighed 5 grams, then the zeolite catalyst weight is reduced 5 weight percent based upon a total weight of the initial zeolite catalyst and templates.

The present invention provides that reducing templates of the zeolite catalyst provides a reduced template zeolite catalyst. The reduced template zeolite catalyst has from 3 weight percent to 15 weight percent of templates maintained following calcination of the zeolite catalyst, based on total weight of zeolite catalyst and remaining templates. In other words, the present invention provides that not all of the templates are removed by calcination to provide the reduced template zeolite catalyst. All individual values and subranges from 3 weight percent to 15 weight percent are included; for example, the reduced template zeolite catalyst can have a lower limit of 3, 4, or 5 weight percent to an upper limit of 15, 14, 13, or 12 weight percent of templates maintained following calcination of the zeolite catalyst.

The present invention is directed towards methods of etherification. Etherification refers to a chemical process, e.g., chemical reaction, that produces ethers. The methods disclosed herein include contacting the reduced template zeolite catalyst with an olefin and an alcohol to produce a monoalkyl ether.

As used herein, "olefin" refers to a compound that is a hydrocarbon having one or more carbon-carbon double bonds. Embodiments of the present disclosure provide that the olefin includes from 6 to 30 carbon atoms. All individual values and subranges from 6 to 30 carbon atoms are included; for example, the olefin can include a lower limit of 6, 8, or 10 carbons to an upper limit of 30, 20, or 14 carbons.

The olefin may include alkenes such as alpha (α) olefins, internal disubstituted olefins, or cyclic structures (e.g., C₃-C₁₂ cycloalkene). Alpha olefins include an unsaturated bond in the α-position of the olefin. Suitable α olefins may be selected from the group consisting of propylene, 1-butene, 1-hexene, 4-methyl-1-pentene, 1-heptene, 1-octene, 1-decene, 1-dodecene, 1-tetradecene, 1-hexadecene, 1-octadecene, 1-icosene, 1-docosene and combinations thereof. Internal disubstituted olefins include an unsaturated bond not in a terminal location on the olefin. Internal olefins may be selected from the group consisting of 2-butene, 2-pentene, 2-hexene, 3-hexene, 2-heptene, 3-heptene, 2-octene, 3-octene, 4-octene, 2-nonene, 3-nonene, 4-nonene, 2-decene, 3-decene, 4-decene, 5-decene and combinations thereof. Other exemplary olefins may include butadiene and styrene.

Examples of suitable commercially available olefins include NEODENE^{™} 6-XHP, NEODENE^{™} 8, NEODENE^{™} 10, NEODENE^{™} 12, NEODENE^{™} 14, NEODENE^{™} 16, NEODENE^{™} 1214, NEODENE^{™} 1416, NEODENE^{™} 16148 from Shell, The Hague, Netherlands.

Embodiments of the present disclosure provide that the alcohol may comprise two hydroxyl groups, i.e., a glycol. The alcohol may include 2 carbons or greater, or 3 carbons or greater, or 4 carbons or greater, or 5 carbons or greater, or 6 carbons or greater, or 7 carbons or greater, or 8 carbons or greater, or 9 carbons or greater, while at the same time, 10 carbons or less, or 9 carbons or less, or 8 carbons or less, or 7 carbons or less, or 6 carbons or less, or 5 carbons or less, or 4 carbons or less, or 3 carbons or less. The alcohol may be selected from the group consisting of monoethylene glycol, diethylene glycol, propylene glycol, triethylene glycol, polyethylene glycol, monopropylene glycol, dipropylene glycol, tripropylene glycol, polypropylene glycol, 1,3-propanediol, 1,2-butanediol, 2,3-butanediol, 1,4-butanediol, 1,6-hexanediol, 1,4-cyclohexanemethanediol, glycerol and, combinations thereof. One or more embodiments provide that the alcohol is selected from the group consisting of monoethylene glycol, diethylene glycol, glycerol, and combinations thereof. The present invention provides that the alcohol is a (poly)alkylene glycol such as monoethylene glycol, diethylene glycol, propylene glycol, or triethylene glycol. Examples of (poly)alkylene glycols include monoethylene glycol, diethylene glycol, triethylene glycol, polyethylene glycol, monopropylene glycol, dipropylene glycol, tripropylene glycol, polypropylene glycol, 1,3-propane diol, 1,2-butane diol, 2,3-butane diol, 1,4-butane diol, 1,6-hexane diol, paraxylene glycol, glycerol, and 1,4-cyclohexane methane diol. One or more embodiments provide that the (poly)alkylene glycol is monoethylene glycol.

Embodiments of the present disclosure provide that the alcohol and the olefin are reacted at a molar ratio of 0.05:1 to 20:1 moles of alcohol to moles of olefin. All individual values and subranges from 0.05:1 to 20:1 are included; for example, the alcohol and the olefin can be reacted at lower limit of 0.05:1, 0.075:1, or 0.1:1 to an upper limit of 20:1, 18:1, or 15:1 moles of alcohol to moles of olefin.

As mentioned, methods disclosed herein include contacting the reduced template zeolite catalyst with an olefin and an alcohol to produce a monoalkyl ether. The olefin and the alcohol may contact the reduced template zeolite catalyst under known etherification conditions and may utilize know reaction equipment and known reaction components. For instance, the olefin and the alcohol may contact the reduced template zeolite catalyst in a slurry reactor, a fixed-bed reactor, or a fluidized-bed reactor. The reactor may operate in batch mode or continuous mode. The reduced template zeolite catalyst may be utilized in an amount such that the reduced template zeolite catalyst is from 1% to 50% by weight based upon a total weight of the olefin, for instance. The olefin and the alcohol may contact the reduced template zeolite catalyst at a reaction temperature from 80 °C to 200 °C, or 100 °C to 150 °C. The reaction pressure may vary for different applications. For instance, the reaction pressure may be a reduced pressure, an atmospheric pressure, or an increased pressure.

Contacting the reduced template zeolite catalyst with the olefin and the alcohol produces a monoalkyl ether. Various monoalkyl ethers may be produced for different applications, e.g., by varying which olefin is utilized and/or by varying which alcohol is utilized. Monoalkyl ethers are utilized for a number of applications such as solvents, surfactants, and chemical intermediates, for instance. Advantageously, the methods of etherification disclosed herein can provide an improved, i.e. greater, monoalkyl ether selectivity, as compared to etherifications that do not utilize the reduced template zeolite catalyst as described herein.

Additionally, the methods of etherification disclosed herein can provide an improved, i.e. lesser, dialkyl ether selectivity, as compared to etherifications that do not utilize the reduced template zeolite catalyst as described herein.

The methods of etherification disclosed herein can provide a monoether selectivity of greater than 85% at an olefin conversion from 0.1 % to 50 %. For instance, the monoether selectivity may be greater than 86%, 87%, or 88% at an olefin conversion from 0.1 % to 50 %.

Surprisingly, the improved monoalkyl ether selectivity and the improved dialkyl ether selectivity, according to embodiments disclosed herein, are not achieved by zeolite catalyst impregnation with a compound analogous, i.e. the same as, to the template. In other words, a zeolite catalyst that does not include templates that is subsequently impregnated with a compound analogous to the template does not provide the improved monoalkyl ether selectivity and the improved dialkyl ether selectivity achieved when utilizing the reduced template zeolite catalyst, as discussed herein.

### EXAMPLES

In the Examples, various terms and designations for materials are used including, for instance, the following:

Zeolite beta catalyst (CP 814E, CAS No. 1318-02-1; SiO₂/Al₂O₃ mole ratio of 25:1; mean pore diameter 6.7 angstroms; surface area 680 m²/g; all organic templates were removed by commercial supplier prior to receipt; obtained from Zeolyst International);

Zeolite beta catalyst (CP 806EL, CAS No. 1318-02-1; SiO₂/Al₂O₃ mole ratio of 25:1; mean pore diameter 6.7 angstroms; surface area 177 m²/g; including organic templates as obtained from commercial supplier; obtained from Zeolyst International).

Thermogravimetric Analysis (TGA) was utilized to determine weight percentage reduction, i.e. a percentage of weight lost due to calcination based upon the initial total weight of the zeolite beta catalyst and the included templates, for zeolite beta catalyst (CP 806EL, including templates as obtained) as follows. The zeolite beta catalyst was calcined at 800 °C in an air environment with a ramping rate of 10 °C/min from starting temperature of 28 °C, where the weight of the zeolite beta catalyst was measured during calcination. Additionally, the weight percent of templates maintained following calcination was also calculated based on TGA experiments for the reduced template zeolite catalysts. For the TGA analysis where the weight loss up to 110 °C was attributed to the removal of adsorbed water and the weight loss up to 110 °C was not attributed to the reduction of templates in the reduced template zeolite catalysts; the templates were assumed to be completely removed, i.e., 100% template reduction, at 800 °C. The weight percent of templates maintained following various calcinations was calculated by: (weight of the catalyst at 110 °C - weight of the catalyst at 800 °C)/(weight of the catalyst at 110 °C) x 100. Determinations were similarly made for the reduced template zeolite catalysts obtained from various calcination conditions herein. The results are reported herein.

Example 1 was performed as follows. Zeolite beta catalyst (CP 806EL; approximately 30 grams) was calcined at 350 °C in an air environment for 8 hours to provide a reduced template zeolite beta catalyst having a 5.6 percentage of weight lost from calcination, based upon a total weight of the initial uncalcined zeolite beta catalyst including templates; the reduced template zeolite beta catalyst had a 8.7 weight percent of templates maintained following calcination of zeolite beta catalyst (based on total weight of zeolite and remained templates). Etherification was performed as follows. The reduced template zeolite beta catalyst (0.75 grams) was added to a vial reactor (40 mL) with rare earth magnetic stir bars (Part #: VP 772FN-13-13-150, V&P Scientific, Inc.); 1-dodecene (6.2 grams) and monoethylene glycol (6.7 grams) were added to the vial reactor; the contents of the vial reactor were heated to 125 °C and stirred for 3 hours for the etherification. Then the contents of the vial reactor were analyzed by gas chromatography. The gas chromatography samples were prepared by adding contents of the vial reactor (100 µL) to 10 mL of internal standard solution (1 mL of hexadecane dissolved in 1 L of ethyl acetate) and were then analyzed offline with an Agilent GC

(7890). For the analysis, dioxane, 1-dodecene (1-C₁₂) and isomers thereof (C₁₂), 2-dodecanol, diethylene glycol, monoalkyl ether and isomers thereof, and dialkyl ether and isomers thereof were included for product quantification such that the weight percent of species of interests were obtained (1-dodecene derived species, which includes monoalkyl ether, dialkyl ether and 2-dodecanol, total amount of dodecene, which includes 1-dodecene and all non 1-dodecene other C₁₂ isomers).

Dodecene derived species were monoether, diether, and 2--dodcanol.
Total amount of dodecene derived species = monoether moles + 2x diether moles + 2 dodecanol;
Total amount of dodecane includes 1-dodecene and all non 1-dodecene other C₁₂ isomers;
Dodecyl-monoether (ME) selectivity (%) was determined as: [total amount of ME]/[total amount of C₁₂ derived species] x 100%.

Dodecyl-diether (DE) selectivity (%) was determined as: 2x[total amount of DE]/[total amount of C₁₂ derived species] x 100%.

Olefin conversion (%) was determined as: [total amount of C₁₂ derived species]/[total amount of C₁₂ derived species + total amount of dodecene] x 100%.

Dodecyl-monoether (ME) yield (%) was determined as: C₁₂ conversion x dodecyl-monoether selectivity.

The results are reported in Table 1.

Examples 2-3 were performed as Example 1 with any changes indicated in Table 1.

Comparative Example A was performed as Example 1 with the change that zeolite beta catalyst (CP 814E) was utilized rather than zeolite beta catalyst (CP 806EL); the zeolite beta catalyst was calcined for 12 hours; any other changes are indicated in Table 1.

**Table 1**

| | Example 1 | Example 2 | Example 3 | Comparative Example A |
|---|---|---|---|---|
| Calcination temperature | 350 °C | 350 °C | 400 °C | 550 °C |
| Calcination environment | air | nitrogen | air | air |
| Weight percent of zeolite beta catalyst lost due to calcination (based upon a total weight of the zeolite catalyst and templates) | 6.2 | Not determined | 8.2 | 15.5 |
| Weight percent of templates maintained following calcination of zeolite beta catalyst | 8.7 | 9.5 | Not determined | 0 |
| Monoalkyl ether selectivity (%) | 95.4 | 98.4 | 97.5 | 81.4 |
| Dialkyl ether selectivity | 2.2 | 1.6 | 2.5 | 18.1 |
| (%) | | | | |
| Olefin conversion (%) | 14.2 | 11.2 | 15.2 | 15.4 |
| Monoalkyl ether yield (%) | 13.5 | 11.0 | 14.8 | 12.5 |

The data of Table 1 illustrate that each of Examples 1-3 had an improved, i.e. greater, monoalkyl ether selectivity and monoalkyl ether yield as compared to Comparative Example A.

The data of Table 1 illustrate that each of Examples 1-3 had an improved, i.e. lesser, dialkyl ether selectivity as compared to Comparative Example A.

Examples 4-7 were performed as Example 1 with the change that the contents of the respective vial reactors were heated to 150 °C rather than 125 °C for the etherification; Example 7 was stirred for 1.5 hours rather than 3 hours for the etherification; any further changes are indicated in Table 2. The results are reported in Table 2.

Comparative Example B performed as Example 1 with the change that the contents of the vial reactor were heated to 150 °C rather than 125 °C for the etherification; Comparative Example B was stirred for 1.0 hours rather than 3 hours for the etherification; any further changes are indicated in Table 2. The results are reported in Table 2.

**Table 2**

| | Example 4 | Example 5 | Example 6 | Example 7 | Comparative Example B |
|---|---|---|---|---|---|
| Calcination temperature | 400 °C | 475 °C | 500 °C | 500 °C | 550 °C (8 hours), then 575 °C (24 hours) |
| Calcination environment | air | air | air | air | air |
| Weight percent of zeolite beta | 8.2 | 14.2 | 14.5 | 14.5 | 16.2 |
| catalyst lost due to calcination (based upon a total weight of the zeolite catalyst and templates) | | | | | |
| Weight percent of templates maintained following calcination of zeolite beta catalyst | Not determined | 7.3 | 5.0 | 5.0 | 0 |
| Monoalkyl ether selectivity (%) | 96.6 | 90.0 | 89.5 | 88.6 | 84.0 |
| Dialkyl ether selectivity (%) | 3.4 | 10.0 | 9.6 | 10.1 | 16.0 |
| Olefin conversion (%) | 29.4 | 35.0 | 34.7 | 34.1 | 32.4 |
| Monoalkyl ether yield (%) | 28.4 | 31.5 | 31.1 | 30.2 | 27.2 |

The data of Table 2 illustrate that each of Examples 4-7 had an improved, i.e. greater, monoalkyl ether selectivity as compared to Comparative Example B.

The data of Table 2 illustrate that each of Examples 4-7 had an improved, i.e. lesser, dialkyl ether selectivity as compared to Comparative Example B.

Example 8 was performed as Example 1 with the change that the contents of the vial reactor were heated to 150 °C rather than 125 °C for the etherification; Example 8 was stirred for 1.0 hours rather than 3 hours for the etherification; any further changes are indicated in Table 3. The results are reported in Table 3.

Comparative Example C was performed as follows. Zeolite beta catalyst (CP806 EL) was calcined at 550 °C in an air environment for 12 hours to remove templates (tetraethylammonium hydroxide) that were located in the micropores of the zeolite beta catalyst; then the zeolite beta catalyst (6.05 grams) was impregnated with tetraethylammonium hydroxide (18.35 grams, 35% aqueous tetraethylammonium hydroxide solution) via stirring in a container for 10 minutes; then the zeolite beta catalyst was dried in a box oven at 100 °C for 1 hour followed by calcination at 400 °C in an air environment for 8 hours. Etherification was performed as Example 1 with the change that the contents of the vial reactor were heated to 150 °C rather than 125 °C for the etherification and reaction time for 1 hour, and 0.35 grams of zeolite beta catalyst was utilized rather than 0.75 grams. The results are reported in Table 3.

**Table 3**

| | Example 8 | Comparative Example C |
|---|---|---|
| Calcination temperature | 400 °C | See description |
| Calcination environment | air | air |
| Weight percent of zeolite beta catalyst lost due to calcination (based upon a total weight of the zeolite catalyst and templates) | 8.2 | Not applicable |
| Weight percent of templates maintained following calcination of zeolite beta catalyst | Not determined | 4.0 weight percent increase due to impregnated templates |
| Monoalkyl | 96.6 | 79.0 |
| ether selectivity (%) | | |
| Dialkyl ether selectivity (%) | 3.4 | 21.0 |
| Olefin conversion (%) | 29.4 | 27.0 |
| Monoalkyl ether yield (%) | 28.4 | 27.0 |

The data of Table 3 illustrate that Example 8 had an improved, i.e. greater, monoalkyl ether selectivity as compared to Comparative Example C.

The data of Table 3 illustrate that Example 8 had an improved, i.e. lesser, dialkyl ether selectivity as compared to Comparative Example C.

The data of Table 3 illustrate that the improved monoalkyl ether selectivity and the improved dialkyl ether selectivity are not achieved via zeolite beta catalyst impregnation with a compound, i.e. tetraethylammonium hydroxide, analogous to the template.

## Claims

1. A method of etherification, the method comprising:
reducing templates of a zeolite catalyst to provide a reduced template zeolite catalyst having from 3 to 15 weight percent of templates maintained following calcination of the zeolite catalyst; and
contacting the reduced template zeolite catalyst with an olefin and an alcohol to produce a monoalkyl ether;
wherein the zeolite catalyst is a zeolite beta catalyst, and wherein the alcohol is a (poly)alkylene glycol.

2. The method of claim 1, wherein a weight of the zeolite catalyst is reduced from 5 weight percent to 15 weight percent based upon a total weight of the zeolite catalyst and templates.

3. The method of any one of claims 1-2, wherein reducing templates of the zeolite catalyst includes calcining the zeolite catalyst at a temperature from 300 °C to 510 °C.

4. The method of claim 3, wherein the zeolite catalyst is calcined from 1 hour to 24 hours.

5. The method of any one of claims 1-4, wherein the templates comprise ammonium ions.

6. The method of any one of claims 1-5, wherein the olefin includes from 6 to 30 carbon atoms.

7. The method of any one of claims 1-6, wherein the olefin is a C₁₂-C₁₄ olefin.

8. The method of any one of claims 1-7, wherein the alcohol is selected from the group consisting of monoethylene glycol, diethylene glycol, and combinations thereof.

## Patentansprüche

1. Verfahren zum Verethern, das Verfahren umfassend:
Reduzieren von Templaten eines Zeolithkatalysators, um einen reduzierten Template-Zeolithkatalysator bereitzustellen, bei dem 3 bis 15 Gewichtsprozent Template nach der Kalzinierung des Zeolithkatalysators erhalten bleiben; und
Kontaktieren des reduzierten Templat-Zeolithkatalysators mit einem Olefin und einem Alkohol, um einen Monoalkylether herzustellen;
wobei der Zeolithkatalysator ein Zeolith-Beta-Katalysator ist und wobei der Alkohol ein (Poly)alkylenglykol ist.

2. Verfahren nach Anspruch 1, wobei das Gewicht des Zeolithkatalysators um 5 Gewichtsprozent auf 15 Gewichtsprozent, bezogen auf das Gesamtgewicht des Zeolithkatalysators und der Templaten, reduziert wird.

3. Verfahren nach einem der Ansprüche 1 bis 2, wobei das Reduzieren der Vorlagen des Zeolithkatalysators das Kalzinieren des Zeolithkatalysators bei einer Temperatur von 300 °C bis 510 °C umfasst.

4. Verfahren nach Anspruch 3, wobei der Zeolithkatalysator von 1 Stunde bis 24 Stunden lang kalziniert wird.

5. Verfahren nach einem der Ansprüche 1 bis 4, wobei die Templaten Ammoniumionen umfassen.

6. Verfahren nach einem der Ansprüche 1 bis 5, wobei das Olefin von 6 bis 30 Kohlenstoffatome einschließt.

7. Verwendung nach einem der Ansprüche 1 bis 6, wobei das Olefin ein C₁₂-C₁₄-Olefin ist.

8. Verfahren nach einem der Ansprüche 1 bis 7, wobei der Alkohol ausgewählt ist aus der Gruppe bestehend aus Monoethylenglykol, Diethylenglykol und Kombinationen davon.

## Revendications

1. Procédé d'éthérification, le procédé comprenant :
la réduction de matrices d'un catalyseur zéolitique pour fournir un catalyseur zéolitique à matrices réduites ayant de 3 à 15 pour cent en poids de matrices conservées à la suite d'une calcination du catalyseur zéolitique ; et
la mise en contact du catalyseur zéolitique à matrices réduites avec une oléfine et un alcool pour produire un éther monoalkylique ;
dans lequel le catalyseur zéolitique est un catalyseur zéolitique bêta, et dans lequel l'alcool est un (poly)alkylène glycol.

2. Procédé selon la revendication 1, dans lequel un poids du catalyseur zéolitique est réduit de 5 pour cent en poids à 15 pour cent en poids en fonction d'un poids total du catalyseur zéolitique et des matrices.

3. Procédé selon l'une quelconque des revendications 1 à 2, dans lequel la réduction des matrices du catalyseur zéolitique comporte la calcination du catalyseur zéolitique à une température allant de 300 °C à 510 °C.

4. Procédé selon la revendication 3, dans lequel le catalyseur zéolitique est calciné de 1 heure à 24 heures.

5. Procédé selon l'une quelconque des revendications 1 à 4, dans lequel les matrices comprennent des ions ammonium.

6. Procédé selon l'une quelconque des revendications 1 à 5, dans lequel l'oléfine comporte de 6 à 30 atomes de carbone.

7. Procédé selon l'une quelconque des revendications 1 à 6, dans lequel l'oléfine est une oléfine en C₁₂ à C₁₄.

8. Procédé selon l'une quelconque des revendications 1 à 7, dans lequel l'alcool est choisi dans le groupe constitué par monoéthylène glycol, diéthylène glycol, et combinaisons de ceux-ci.
